# EUROPÄISCHE PATENTSCHRIFT

(11) **EP 0 571 405 B1**
(45) Veröffentlichungstag und Bekanntmachung des Hinweises auf die Patenterteilung: **01.05.1996**
(21) Anmeldenummer: 92903077.3
(22) Anmeldetag: 31.01.1992
(51) Int. Cl.: C07C 317/14, C07C 313/04

(54) **Verfahren zur Herstellung von 4-Alkylsulfonyl-1-alkyl-2-chlorbenzolen und Zwischenprodukte**
Process for preparing 4-alkylsulfonyl-1-alkyl-2-chlorobenzenes and intermediates
Procédé de préparation de 4-alkylsulfonyl-1-alkyl-2-chlorobenzols et des produits intermédiaires

(30) Priorität: 14.02.1991 DE 4104393
(43) Veröffentlichungstag der Anmeldung: 01.12.1993
(73) Patentinhaber: HOECHST AKTIENGESELLSCHAFT, D-65926 Frankfurt am Main (DE)
(72) Erfinder: FOLZ, Georg, D-6000 Frankfurt am Main 71 (DE); PAPENFUHS, Theodor, D-6000 Frankfurt am Main 50 (DE)
(86) Internationale Anmeldenummer: EP9200205
(87) Internationale Veröffentlichungsnummer: WO9214700

(56) Entgegenhaltungen:
- EP-A- 0 115 328
- EP-A- 0 258 190
- WO-A-91/07384
- DE-C- 133 000
- US-A- 4 012 442
- US-A- 4 675 447
- F. MUTH IN HOUBEN-WEYL, METHODEN DER ORGANISCHEN CHEMIE , BAND IX , 4. AUFL. SEITE 301 - SEITE 342 'METHODEN ZUR HERSTELLUNG UND UMWANDLUNG VON AROMATISCHEN SULFINSÄUREN' 1955 , E. MÜLLER , GEORG THIEME VERLAG, STUTTGART, DE

## Beschreibung

Die Erfindung betrifft ein Verfahren zur Herstellung von 4-Alkyl (C₁-C₄)-sulfonyl-1-alkyl (C₁-C₄)-2-chlorbenzolen, insbesondere von 4-Methylsulfonyl-1-methyl-2-chlorbenzol, wobei die bei diesem Verfahren als Zwischenprodukte gebildeten 4-Alkyl(C₁-C₄)-3-chlor-benzolsulfinsäuren neue Verbindungen darstellen.

Die Verbindungen sind wertvolle Zwischenprodukte für Pesticide und Herbicide.

Es ist bekannt, 4-Alkylsulfonyl-1-alkyl-2-chlorbenzole herzustellen, indem man 4-Alkylsulfonylalkylbenzole mit Sulfurylchlorid in Gegenwart von Antimontrichlorid als Katalysator chloriert (US-PS 4 675 447).

Nachteilig ist hierbei der Einsatz von relativ teurem Sulfurylchlorid, verbunden mit einem erheblichen Anfall von umweltbelastendem Schwefeldioxid und die Bereitstellung von isolierten, trockenen 4-Alkylsulfonylalkylbenzolen.

Es wurde nun überraschenderweise gefunden, daß man 4-Alkylsulfonyl-1-alkyl-2-chlorbenzole der allgemeinen Formel (4) 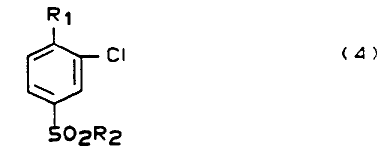 in welcher R₁, R₂ gleiche oder verschiedene Alkylgruppen von 1 bis 4 Kohlenstoffatomen bedeuten, unter Vermeidung der mit dem zum Stand der Technik zählenden Verfahren verbundenen Nachteile in sehr guten Ausbeuten und hoher Selektivität herstellen kann, indem man 1 Mol eines p-Alkylbenzolsulfonsäurechlorids der allgemeinen Formel (1) 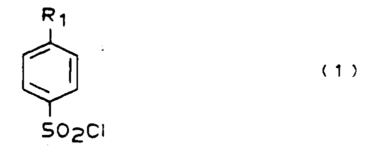 in welcher R₁ die vorstehend genannte Bedeutung hat, mit mindestens 1 Mol gasförmigem Chlor in Gegenwart eines Chlorüberträgers, wie beispielsweise Eisen(III)-chlorid oder Eisen(III)-chlorid und Iod, bei Temperaturen von etwa 50°C bis etwa 100°C, vorzugsweise von etwa 70°C bis etwa 80°C, selektiv chloriert zur Verbindung der allgemeinen Formel (2) 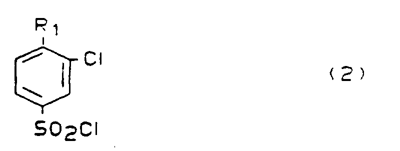 in welcher R₁ die vorstehend genannte Bedeutung hat, diese ausschließlich in wäßrigem Medium bei pH-Werten von etwa 8 bis 10 mit 1 bis etwa 1,2 Mol, vorzugsweise 1,1 Mol, Natriumhydrogensulfit oder Natriumsulfit bei Temperaturen von etwa 40 bis etwa 90°C, vorzugsweise von etwa 50 bis etwa 65°C, zur Verbindung der allgemeinen Formel (3) 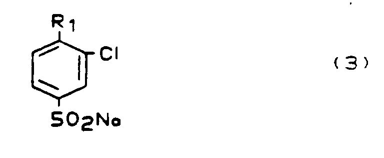 in welcher R₁ die vorstehend genannte Bedeutung hat, reduziert und diese mit 1 bis etwa 2,2 Mol, vorzugsweise etwa 1,25 bis etwa 1,90 Mol, besonders bevorzugt etwa 1,50 bis etwa 1,75 Mol Alkyl(C₁-C₄)-chlorid, in Gegenwart eines säurebindenden Mittels, vorzugsweise Magnesiumoxid, zur Verbindung der genannten allgemeinen Formel (4) bei Temperaturen von etwa 80 bis etwa 150°C umsetzt.

Das erfindungsgemäße Verfahren wird im einzelnen wie folgt durchgeführt.

### a) Chlorierung:

Die Chlorierung der p-Alkyl(C₁-C₄)-benzolsulfonsäurechloride wird vorteilhaft in Substanz in Abwesenheit von Lösungsmitteln durch Einleiten von elementarem Chlor beetwa 50 - 100°C, vorzugsweise bei 70 - 80°C unter Einsatz von etwa 0,1 bis etwa 1 %, vorzugsweise etwa 0,5 %, eines Katalysatorgemischs aus 75 % Eisen(III)-chlorid und 25 % Iod, durchgeführt. Die Chlorierung wird bei einem Einchlorierungsgrad von ca. 100 % abgebrochen. (Endpunktbestimmung über GC). Das angefallene Produkt enthält neben Spuren von Ausgangsverbindung nur ganz geringe Mengen der Dichlorverbindung. Das Chlorierungsprodukt kann, wie es anfällt, zur Weiterbearbeitung eingesetzt werden; es kann aber auch gewaschen, sowie destilliert oder franktioniert werden. Die Ausbeute an 3-Chlor-4-alkyl(C₁-C₄)-benzolsulfonsäurechloriden ist praktisch quantitativ.

### b) Reduktion:

Die Reduktion der gemäß a) erhaltenen 2-Chlor-4-alkyl (C₁-C₄)-benzolaulfonsäurechloride zu den 4-Alkyl(C₁-C₄)-3-chlor-benzolsulfinsäuren kann in an sich bekannter Weise erfolgen (vgl. Houben-Weyl, Bd. 9, S. 304-311). Vorteilhaft führt man die Reduktion mit NaHSO₃ + NaOH, bevorzugt mit Natriumsulfit + NaOH durch. Die Reduktion läßt sich am besten in wäßrigem Medium durchführen, indem man eine Lösung von Natriumsulfit vorlegt und gleichzeitig 2-Chlor-4-alkylbenzolsulfonsäurechlorid (in aufgeschmolzenem Zustand) sowie Natronlauge (handelsübliche 35 %ige) unter pH-Kontrolle von etwa 8 bis etwa 10 zutropft. Man kann auch das Sulfonsäurechlorid zur Sulfitlösung geben und die Natronlauge unter Aufrechterhaltung eines pH von etwa 8 bis etwa 10 zugeben. Die Konzentration der Sulfitlösung kann zwischen etwa 10 und etwa 30 % schwanken, wobei eine etwa 15 %ige Lösung zu bevorzugen ist. Die Sulfitmenge bewegt sich zwischen der theoretischen Menge und einem Überschuß von etwa 20 %, wobei ein Überschuß von etwa 10 % am günstigsten ist. Die Reaktion läuft bei einer Temperatur von etwa 40 bis etwa 90°C, bevorzugt zwischen etwa 50 und etwa 65°C ab. Die Reduktion ist beendet, wenn der pH-Wert von etwa 8 bis etwa 10 konstant bleibt. Aus der Lösung kristallisiert bei etwa 45°C das Natriumsalz der 4-Alkyl(C₁-C₄)-3-chlor-benzolsulfinsäure aus. Die Verbindung kann ohne Zwischenisolierung weiterverarbeitet werden.

### c) Alkylierung:

Die Alkylierung der gemäß b) erhaltenen 4-Alkyl(C₁-C₄)-3-chlorbenzolsulfinsäuren (Na-Salze) zu den entsprechenden 4-Alkyl(C₁-C₄)-sulfonyl-2-chlor-alkylbenzolen findet mit Alkyl(C₁-C₄)-chloriden, bevorzugt mit Methylchlorid, statt. Für diese Reaktion wird die gemäß b) angefallene Reduktionalösung (eine Isolierung des Sulfinats ist nicht erforderlich) in einen Druckapparat (Autoklav) gegeben und mit den entsprechenden Alkyl(C₁-C₄)-chloriden und einem säurebindenden Mittel, vorzugsweise Magnesiumoxid, zur Neutralisation des bei der Verseifung der überschüssigen Alkyl(C₁-C₄)-chlorids entstehenden Chlorwasserstoffs, versetzt. Der Einsatz der Alkyl(C₁-C₄)-chloride bewegt sich im Bereich von etwa 100 bis etwa 220 % der Theorie, vorteilhaft zwischen etwa 125 bis etwa 190 %, besonders bevorzugt zwischen etwa 150 und etwa 175 %. Die Alkylchloride können auf einmal zugesetzt oder während der Reaktion in Anteilen oder kontinuierlich zugegeben werden. Die Reaktionstemperaturen liegen bei der Methylierung bei etwa 80 bis etwa 100°C. Bei der Anwendung von Alkyl(C₄)-chloriden ist eine Reaktionstemperatur von etwa 150°C erforderlich.

Als säurebindende Mittel können außer Magnesiumoxid beispielsweise Lithium-, Natrium-, Kalium-, Rubidium-, Caesium- , Magnesium-, Calcium- , Barium- oder Strontiumhydroxid, -carbonat oder -hydrogencarbonat oder Mischungen daraus verwendet werden.

Die nachfolgenden Beispiele sollen das erfindungsgemäße Verfahren näher erläutern, ohne es darauf zu beschränken.

### Beispiel 1 (Chlorierung)

### Herstellung von 2-Chlor-p-toluolsulfonsäurechlorid

In einem Chlorierungsapparat werden 576 g (3 mol) p-Toluolsulfonsäurechlorid aufgeschmolzen. Nach Zugabe von 7 g Eisen(III)-chlorid und 2 g Iod wurde bei 75 - 80°C so lange elementares Chlor eingeleitet, bei einer Chlorbelastung von 5 l Cl₂/h, bis praktisch 100 %ige Einchlorierung erfolgt war (GC-Analyse).

Das Produkt kann nach Ausblasen von Restchlor und HCl roh eingesetzt werden; es kann jedoch auch gewaschen und, wenn erwünscht, destilliert werden.

Die Ausbeute beträgt etwa 100 % d. Th.
Reinheit: ≥ 97 % (neben wenig Ausgangsverbindung und Dichlorverbindung)

### Beispiel 2 (Reduktion)

### Herstellung von 4-Methyl-3-chlor-benzolsulfinsäure (Na-Salz)

In einem mit 2 Tropfrichtern und einer pH-Elektrode versehenen Rührapparat werden 2350 ml Wasser und 420 g (3,3 mol) Natriumsulfit (= 15,1 % Na₂SO₃-Lösung) vorgelegt und auf 50 - 55°C hochgeheizt. Im Verlauf von etwa 2 h läßt man 677 g (3 mol) 2-Chlor-p-toluolsulfonsäurechlorid einlaufen und gleichzeitig 690 g NaOH (35 %ig) so zutropfen, daß stets ein pH von 8 - 10 gehalten wird. Die Temperatur darf dabei bis 65°C steigen. Die Reaktion ist beendet, wenn der pH-Wert von 8 - 10 konstant bleibt. Das Natriumsulfinat ist bei 60 - 65°C gelöst und kristallisiert ab etwa 55°C aus.

Das so erhaltene Produkt wird zweckmäßigerweise ohne Zwischenisolierung in die nächste Stufe (Alkylierung) eingesetzt.

### Beispiel 3 (Alkylierung)

### Herstellung von 4-Methylsulfonyl-1-methyl-2-chlorbenzol

Die gemäß Beispiel 2 angefallene Lösung der 4-Methyl-3-chlor-benzolsulfinsäure (Na-Salz) wird in einem Autoklav vorgelegt und mit 45 g Magnesiumoxid versetzt. Dann wird der Autoklav verschlossen. Anschließend drückt man 270 g (5,3 mol; 175 % d. Th.) Methylchlorid ein, wobei der Druck auf 6 - 7 bar steigt, und heizt innerhalb von etwa 1 h auf 90 - 95°C; bei 80 - 85°C wird ein Maximaldruck von 13 - 14 bar erreicht, der in der Folge wegen Methylchlorid-Verbauchs bis auf 4 - 5 bar sinkt. Es wird dann noch 4 h nachgerührt.

Nach dem Abkühlen wird entspannt und das Reaktionsgemisch mit 225 g Salzsäure (30 %ig) sauer gestellt. Anschließend wird ca. 1 h bei 90 - 95°C gerührt, um alles Magnesiumoxid und eventuelle Nebenprodukte in Lösung zu bringen. Durch Abkühlen wird auskristallisiert und das Produkt durch Filtration isoliert.

Die Ausbeute beträgt etwa 90 % (gerechnet über die Stufen der Reduktion und Methylierung).
Gehalt: ≥ 97 %
Schmelzpunkt: 88 - 91°C

### Beispiel 4 (Alkylierung)

### Herstellung von 4-Ethylsulfonyl-1-methyl-2-chlorbenzol

Die gemäß Beispiel 2 angefallene Lösung der 4-Methyl-3-chlor-benzolsulfinsäure (Na-Salz) wird in einem Autoklav vorgelegt und mit 45 g Magnesiumoxid versetzt. Dann wird der Autoklav verschlossen und 342 g (5,3 mol, 175 % d.Th.) Ethylchlorid eingeleitet, wobei der Druck auf 4,5 bar steigt. Darauf wird innerhalb von 1 h auf 130-140°C erhitzt, wobei ein Maximaldruck von ca. 12 bar erreicht wird, der in der Folge wegen des Ethylenchlorid-Verbrauchs bis auf ca. 5-6 bar sinkt. Anschließend wird noch 4-5 h nachgerührt.

Nach dem Abkühlen wird entspannt und das Reaktionsgemisch mit 225 g Salzsäure (30 %ig) sauergestellt. Anschließend wird ca. 1 h bei 90-95°C gerührt, um alles Magnesiumoxid und eventuelle Nebenprodukte in Lösung zu bringen. Durch Abkühlen wird auskristallisiert und das Produkt durch Filtration isoliert.

Die Ausbeute beträgt ca. 90 % (gerechnet über die Stufen der Reduktion und Ethylierung).
Gehalt: ≥ 97 %
Schmelzpunkt: 50-52°C

| | | | | |
|---|---|---|---|---|
| C₉H₁₁ClO₂S | ber.: | C 49,38 %; | H 5,029 %; | Cl 16,23 % |
| | gef. : | C 49,19 %; | H 5,014 %; | Cl 16,37 % |

### Beispiel 5 (Chlorierung)

### Herstellung von 3-Chlor-4-ethylbenzolsulfonsäurechlorid

Es wurde, wie in Beispiel 1 beschrieben, gearbeitet, lediglich mit dem Unterschied, daß nun 615 g (3 mol) 4-Ethylbenzolsulfonsäurechlorid eingesetzt wurden. Die Ausbeute betrug etwa 100 % d.Th..
Reinheit: ≥ 96 % (neben wenig Ausgangsverbindung und Dichlorverbindung)
Erstarrungspunkt: 21-22°C
Siedepunkt (2,5-3 mm Hg): 129-130°C.

| | | | | |
|---|---|---|---|---|
| C₈H₈Cl₂Oₛ | ber.: | C 49,18 %; | H 3,37 %; | Cl 29,65 % |
| | gef. : | C 40,02 ; | H 3,29 %; | Cl 29,73 % |

### Beispiel 6 (Reduktion)

### Herstellung von 3-Chlor-4-ethylbenzolsulfinsäure (Na-Salz)

Es wurde, wie in Beispiel 2 beschrieben, gearbeitet, lediglich mit dem Unterschied, daß nun 718 g (3 mol) 3-Chlor-4-ethylbenzolsulfonsäurechlorid eingesetzt wurden.

Das Natriumsulfinat kristallisierte erst bei tiefer Temperatur aus.

Das so erhaltene Produkt wird zweckmäßigerweise ohne Zwischenisolierung in die nächste Stufe (Alkylierung) eingesetzt (vgl. Beispiele 7 und 8; Ausbeute auch dort).

### Beispiel 7 (Alkylierung)

### Herstellung von 4-Methylsulfonyl-1-ethyl-2-chlorbenzol

Die gemäß Beispiel 6 angefallene Lösung der 3-Chlor-4-ethylbenzolsulfinsäure (Na-Salz) wird in einem Autoklav vorgelegt. Anschließend wird, wie in Beispiel 3 beschrieben weiter verfahren.

Die Ausbeute beträgt ca. 88 % d.Th. (gerechnet über die Stufen der Reduktion und Methylierung)
Gehalt: ≥ 96 %
Schmelzpunkt: 58-60°C

| | | | | |
|---|---|---|---|---|
| C₉H₁₁ClO₂S | ber.: | C 49,427%; | H 5,07 %; | Cl 16,211% |
| | gef. : | C 49,29 %; | H 5,03 %; | Cl 16,32 % |

### Beispiel 8 (Alkylierung)

### Herstellung von 4-Ethylsulfonyl-1-ethyl-2-chlorbenzol

Die gemäß Beispiel 6 angefallene Lösung der 3-Chlor-4-ethylbenzolsulfinsäure (Na-Salz) wird in einem Autoklav vorgelegt. Dann wird, wie in Beispiel 4 beschrieben, weiter verfahren. Wegen des niedrigeren Erstarrungspunktes des Produkts wird bei 40-50°C flüssig getrennt (organische Phase unten) und nach Zugabe von ca. 1000 ml Wasser destilliert (1,5 mm Hg, 153-155°C).

Die Ausbeute beträgt etwa 80 % d.Th. (gerechnet über die Stufen der Reduktion und Ethylierung).
Gehalt: ≥ 96 % Schmelzpunkt: 36-37°C ,

| | | | | |
|---|---|---|---|---|
| C₁₀H₁₃ClO₂S | ber.: | C 51,609 %; | H 5,633 %; | Cl 15,233 % |
| | gef. : | C 51,53 %; | H 5,49 %; | Cl 15,37 % |

## Patentansprüche

1. Verfahren zur Herstellung von 4-Alkyl(C₁-C₄)-sulfonyl-1-alkyl-2-chlorbenzolen der allgemeinen Formel (4) 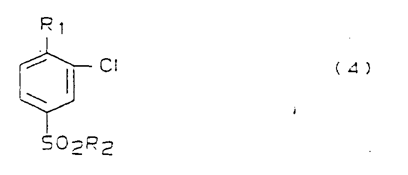 in welcher R₁, R₂ gleiche oder verschiedene Alkylgruppen von 1 bis 4 Kohlenstoffatomen bedeuten, in sehr guten Ausbeuten und hoher Selektivität, dadurch gekennzeichnet, daß man 1 Mol eines p-Alkylbenzolsulfonsäurechlorids der allgemeinen Formel (1) 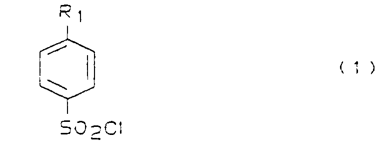 in welcher R₁ die vorstehend genannte Bedeutung hat, mit mimdestens 1 Mol gasförmigem Chlor in Gegenwart eines chlorüberzrägers bei Temperaturen von etwa 50°C bis eta 100°C selektiv chloriert zur Verbindung der allgemeimen Formel (2) 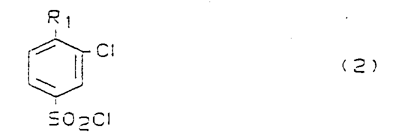 in welcher R₁ die vorstehend genannte Bedeutung hat, diese anschlißend in wäßrigem Medium bei pH-Werten von etwa 8 bis etwa 10 mit 1 bis etwa 1,2 Mol Natriumhydrogensulfit oder Natriumsulfit bei Temperaturen von etwa 40 bis etwa 90 °C zur Verbindung der allgemeinen Formel (3) 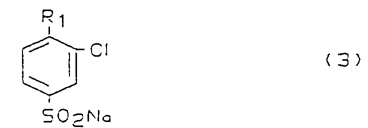 in welcher R₁ die vorstehend genannte Bedeutung hat, reduziert und diese mit 1 bis etwa 2,2 Mol Alkyl(C₁-C₄)-chlorid in Gegenwart eines säurebindenden Mittels bei Temperaturen von etwa 80 bis etwa 150°C zur Verbindung der genannten allgemeinen Formel (4) umsetzt.

2. Verfahrem nach Anspruch 1, dadurch gekennzeichnet, daß man die Chlorierurg bei Temperaturen von etwa 70 bis etwa 80°C vornimmt.

3. Verfahren nach mindestens einem der Ansprüche 1 und 2, dadurch gekennzeichnet, daß man die Chlorierung in Gegenwart von Eisen(III)-chlorid vornimmt.

4. Verfahren nach mindestens einem der Ansprüche 1 und 2, dadurch gekennzeichnet, daß man die Chlorierung in Gegenwart von Eisen (III)-chlorid und Iod vornimmt.

5. Verfahren nach mindestens einem der Ansprüche 1 bis 4, dadurch gekennzeichnet, daß man 1 Mol 3-Chlor-4-alkylbenzolsulfonsäurechlorid mit 1,1 Mol Natriumhydrogensulit oder Natriumsulfit reduziert,

6. Verfahren nach mindestens einem der Ansprüche 1 bis 5, dadurch gekennzeichnet, daß man das 3-Chlor-4-alkylbenzolsulfonsäurechlorid bei Temperaturen von etwa 50 bis etwa 65 °C reduziert.

7. Verfahren nach mindestens einem der Ansprüche 1 bis 6, dadurch gekennzeichnet, daß man 1 Mol des 4-alkyl(C₁-C₄)-3-chlor-benzolsulfinsauren Natriums mit etwa 1,25 bis etwa 1,90 Mol Alkyl(C₁-C₄)-chlorid alkyliert.

8. Verfahren nach mindestens einem der Ansprüche 1 bis 7, dadurch gekennzeichnet, daß man 1 Mol des 4-alkyl(C₁-C₄)-3-chlorbenzolsulfinsauren Natriums mit etwa 1,50 bis etwa 1,75 Mol Alkyl(C₁-C₄)-chlorid alkyliert.

9. Verfahren nach mindestens einem der Ansprüche 1 bis 8, dadurch gekennzeichnet, daß man das 4-alkyl(C₁-C₄)-3-chlorbenzolsulfinsaure Natrium in Gegenwart von Magnesiumoxid als säurebindendem Mittel alkyliert.

10. Verfahren nach mindestens einem der Ansprüche 1 bis 9, dadurch gekennzeichnet, daß man das 4-methyl-3-chlorbenzolsulfinsaure Natrium mit Methylchlorid bei Temperaturen von etwa 80 bis etwa 100°C methyliert.

11. Verbindungen der allgemeinen Formel 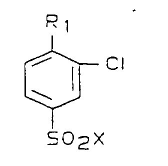 in welcher R₁ eine Alkylgruppen von 1 bis 4 Kohlenstoffatomen und X ein Wasserstoff- oder Alkalimetallatom bedeutet.

## Claims

1. A process for the preparation of a 4-alkyl(C₁-C₄)sulfonyl-1-alkyl-2-cblorobenzene of the formula (4) 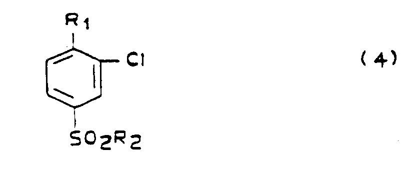 in which R₁ and R₂ are identical or different alkyl groups having 1 to 4 carbon atoms, in very good yields and in high selectivity, which comprises selectively chlorinating 1 mol of a p-alkylbenzenesulfonyl chloride of the formula (1) 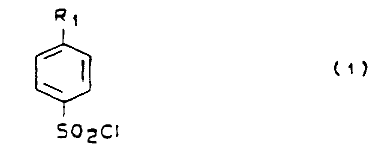 in which R₁ has the meaning given above, with at least 1 mol of gaseous chlorine in the presence of a chlorine carrier at temperatures of about 50°C to about 100°C to give the compound of the formula (2) 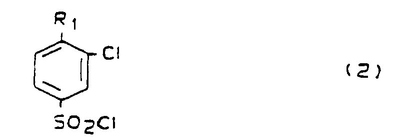 in which R₁ has the meaning given above, subsequently reducing the latter in an aqueous medium at a pH of about 8 to about 10 with 1 to about 1.2 mol of sodium hydrogen sulfite or sodium sulfite at temperatures of about 40 to about 90°C to give the compound of the formula (3) 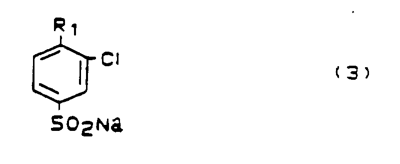 in which R₁ has the meaning given above, and reacting the latter with 1 to about 2.2 mol of alkyl(C₁-C₄) chloride in the presence of an acid binder at temperatures of about 80 to about 150°C to give the compound of the formula (4) mentioned.

2. The process as claimed in claim 1, wherein the chlorination is carried out at temperatures of about 70 to about 80°C.

3. The process as claimed in at least one of claims 1 and 2, wherein the chlorination is carried out in the presence of iron(III) chloride.

4. The process as claimed in at least one of claims 1 and 2, wherein the chlorination is carried out in the presence of iron(III) chloride and iodine.

5. The process as claimed in at least one of claims 1 to 4, wherein 1 mol of 3-chloro-4-alkylbenzenesulfonyl chloride is reduced with 1.1 mol of sodium hydrogen sulfite or sodium sulfite.

6. The process as claimed in at least one of claims 1 to 5, wherein the 3-chloro-4-alkylbenzenesulfonyl chloride is reduced at temperatures of about 50 to about 65°C.

7. The process as claimed in at least one of claims 1 to 6, wherein 1 mol of the sodium 4-alkyl(C₁-C₄)-3-chlorobenzenesulfinate is alkylated using about 1.25 to about 1.90 mol of alkyl(C₁-C₄) chloride.

8. The process as claimed in at least one of claims 1 to 7, wherein 1 mol of the sodium 4-alkyl(C₁-C₄)-3-chlorobenzenesulfinate is alkylated using about 1.50 to about 1.75 mol of alkyl(C₁-C₄) chloride.

9. The process as claimed in at least one of claims 1 to 8, wherein the sodium 4-alkyl(C₁-C₄)-3-chlorobenzenesulfinate is alkylated in the presence of magnesium oxide as an acid binder.

10. The process as claimed in at least one of claims 1 to 9, wherein the sodium 4-methyl-3-chlorobenzenesulfinate is methylated using methyl chloride at temperatures of about 80 to about 100°C.

11. A compound of the formula 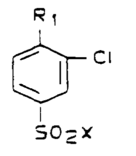 in which R₁ is an alkyl group having 1 to 4 carbon atoms and X is a hydrogen atom or an alkali metal atom.

## Revendications

1. Procédé pour préparer des 4-(alkyle en C₁-C₄)sulfonyl-1-alkyl-2-chlorobenzènes de formule générale (4) 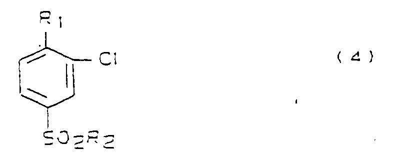 dans laquelle R₁ et R₂ sont des groupes alkyle identiques ou différents, ayant de 1 à 4 atomes de carbone, avec de très bons rendements et une grande sélectivité, caractérisé en ce qu'on soumet à une chloration sélective 1 mole d'un chlorure de p-alkylbenzènesulfonyle de formule générale (1) 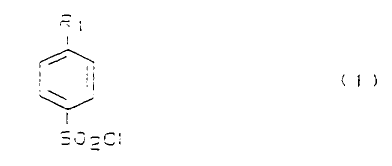 dans laquelle R₁ a la signification donnée ci-dessus, avec au moins 1 mole de chlore gazeux, en présence d'un porteur de chlore, à des températures d'environ 50°C à environ 100°c, pour obtenir le composé de formule générale (2) 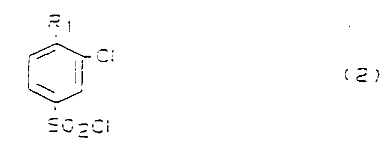 dans laquelle R₁ a la signification donnée ci-dessus ; puis on réduit ce dernier en milieu aqueux à des pH d'environ 8 à environ 10, avec 1 à environ 1,2 mole d'hydrogénosulfite de sodium ou de sulfite de sodium, à des températures d'environ 40 à environ 90°C, pour obtenir le composé de formule générale (3) 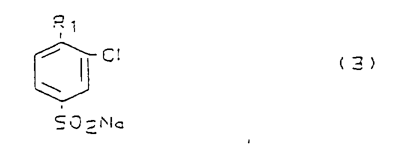 dans laquelle R₁ a la signification donnée ci-dessus ; et on fait réagir ce dernier avec 1 à environ 2,2 moles d'un chlorure d'alkyle en C₁-C₄, en présence d'un agent fixant les acides, à des températures d'environ 80 à environ 150°C, pour obtenir le composé ayant la formule générale (4) mentionnée ci-dessus.

2. Procédé selon la revendication 1, caractérisé en ce qu'on procède à la chloration à des températures d'environ 70 à environ 80°C.

3. Procédé selon au moins l'une des revendications 1 et 2, caractérisé en ce qu'on procède à la chloration en présence de chlorure de fer(III).

4. Procédé selon au moins l'une des revendications 1 et 2, caractérisé en ce qu'on procède à la chloration en présence de chlorure de fer(III) et d'iode.

5. Procédé selon au moins l'une des revendications 1 à 4, caractérisé en ce qu'on réduit 1 mole de chlorure de 3-chloro-4-alkylbenzènesulfonyle avec 1,1 mole d'hydrogénosulfite de sodium ou de sulfite de sodium.

6. Procédé selon au moins l'une des revendications 1 à 5, caractérisé en ce qu'on réduit le chlorure de 3-chloro-4-alkylbenzènesulfonyle à des températures d'environ 50 à environ 65°C.

7. Procédé selon au moins l'une des revendications 1 à 6, caractérisé en ce qu'on alkyle 1 mole du sel de sodium d'un acide 4-(alkyle en C₁-C₄)-3-chlorobenzènesulfinique avec environ 1,25 à environ 1,90 mole de chlorure d'alkyle en C₁-C₄.

8. Procédé selon au moins l'une des revendications 1 à 7, caractérisé en ce qu'on alkyle 1 mole du sel de sodium de l'acide 4-(alkyle en C₁-C₄)-3-chlorobenzènesulfinique avec d'environ 1,50 à 1,75 mole de chlorure d'alkyle en C₁-C₄.

9. Procédé selon au moins l'une des revendications 1 à 8, caractérisé en ce qu'on alkyle le sel de sodium de l'acide 4-(alkyle en C₁-C₄)-3-chlorobenzènesulfinique en présence d'oxyde de magnésium servant d'agent de fixation des acides.

10. Procédé selon au moins l'une des revendications 1 à 9, caractérisé en ce qu'on méthyle le sel de sodium de l'acide 4-méthyl-3-chlorobenzènesulfinique avec du chlorure de méthyle à des températures d'environ 80 à environ 100°C.

11. Composés de formule générale 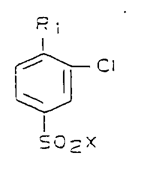 dans laquelle R₁ est un groupe alkyle ayant de 1 à 4 atomes de carbone, et X est un atome d'hydrogène ou d'un métal alcalin.
